# EUROPEAN PATENT SPECIFICATION

(11) **EP 3 606 363 B1**
(45) Date of publication and mention of the grant of the patent: **05.05.2021**
(21) Application number: 18709021.2
(22) Date of filing: 09.03.2018
(51) Int. Cl.: A24F 40/465

(54) **SUSCEPTOR FOR USE WITH AN INDUCTIVELY HEATED AEROSOL-GENERATING DEVICE OR SYSTEM**
SUSZEPTOR ZUR VERWENDUNG MIT EINER INDUKTIV ERWÄRMTEN AEROSOLBILDENDEN VORRICHTUNG ODER SYSTEM
SUSCEPTEUR DESTINÉ À ÊTRE UTILISÉ AVEC UN DISPOSITIF OU UN SYSTÈME DE PRODUCTION D'AÉROSOL CHAUFFÉ PAR INDUCTION

(30) Priority: 05.04.2017 EP 17164907
(43) Date of publication of application: 12.02.2020
(73) Proprietor: Philip Morris Products S.A., 2000 Neuchâtel (CH)
(72) Inventor: COURBAT, Jérôme, 2000 Neuchâtel (CH); ZINOVIK, Ihar Nikolaevich, 2000 Neuchâtel (CH); MIRONOV, Oleg, 2000 Neuchâtel (CH)
(74) Representative: Ponder, William Anthony John
(86) International application number: PCT/EP2018/055971
(87) International publication number: WO 2018/184787

(56) References cited:
- WO-A1-2015/177045
- WO-A1-2015/177252
- WO-A1-2015/177254
- WO-A1-2015/177294
- CN-U- 203 748 673

## Description

The present invention relates to a susceptor for holding and inductively heating an aerosol-forming liquid. The invention further relates to a cartridge for use with an aerosol-generating device as well as to aerosol-generating device and system for generating an aerosol by inductively heating an aerosol-forming liquid.

Aerosol-generating systems based on inductively heating an aerosol-forming substrate are generally known from prior art. These systems may comprise an induction source for generating an alternating electromagnetic field which induces at least one of heat generating eddy currents or hysteresis losses in a susceptor. The thus heated susceptor is in thermal proximity of an aerosol-forming substrate which is capable of releasing volatile compounds to form an aerosol upon heating. Depending on the type of the aerosol-generating system, the susceptor and the aerosol-forming substrate may be provided together in an aerosol-generating article, in particular in a cartridge. The cartridge may be configured to be received in a cavity of an aerosol-generating device which in turn includes the induction source. Several susceptor configurations have been described in the art in order to ascertain an adequate heating of the aerosol-forming substrate. Yet, in many cases the susceptor is in contact only with a small portion of the aerosol-forming substrate. This may lead to an inhomogeneous heating across the substrate volume such that the temperature of the substrate is partially too low to form an aerosol. Consequently, only a small portion of the substrate is effectively utilized for the user experience. Yet, increasing the heating power in order to heat up all portions of the substrate to the required temperature for aerosol formation may cause local overheating of those portions being in direct contact with the susceptor.

WO2015177043 discloses an aerosol-generating system comprising an aerosol-generating device and a cartridge. The cartridge comprises an inductively heatable susceptor element that is fluid permeable, wherein the inductively heatable susceptor element may be soaked in aerosol-forming substrate.

Therefore, it would be desirable to have a susceptor as well as a cartridge and aerosol-generating device comprising a susceptor with the advantages of prior art solutions but without their limitations. In particular, it would be desirable to have a susceptor, a cartridge and aerosol-generating device allowing for a homogenous heating of aerosol-forming substrate without the risk of local overheating.

According to the invention, there is provided an inductively heatable susceptor for use with an aerosol-generating device or system. The susceptor comprises an open-porous inductively heatable ceramic material for holding an aerosol-forming liquid and for heating the liquid under the influence of an alternating electromagnetic field. In particular, the susceptor may be made or consist of this open-porous ceramic material.

The ceramic material according to the invention is characterized on the one hand by its open-porous or open-pored structure and on the other hand by its capability of being heatable under the influence of an alternating electromagnetic field. Due to this, the susceptor is advantageously both, a storage medium for aerosol-forming liquid to be heated as well as a heating element for inductively heating the liquid held therein. For this reason, the susceptor according to the invention may be considered to be a dual function susceptor. Advantageously, the open-porous structure of the ceramic material allows the entire susceptor material to be homogenously soaked with aerosol-forming liquid. Therefore, the susceptor is over all in direct contact with aerosol-forming liquid. At the same time, the entire volume of the susceptor is homogenously heatable under the influence of an alternating electromagnetic field. For this reason, the susceptor according to the invention advantageously allows for homogeneously heating the entire aerosol-forming liquid stored therein without the need to overheat. Furthermore, the susceptor according to the invention advantageously ensures a very consistent user experience because the quantity of aerosol-forming liquid which may be heated is related to the porosity and the overall volume of the susceptor which are well controllable parameters.

The open porosity of susceptor provides high retention capacity for liquid aerosol-forming material. Therefore, liquid aerosol-forming material is safely held or retained in the susceptor. Advantageously, this reduces the risk of spill, for example as compared to a liquid tank. In particular, this allows the susceptor as well as any aerosol-generating articles, devices or system comprising such a susceptor to be leak proof. In addition, the open porosity of the susceptor material allows vaporized aerosol-forming material to freely escape from the cartridge upon heating.

As used herein, the term 'susceptor' refers to an element comprising a material that is capable to convert electromagnetic energy into heat. Thus, when located in an alternating electromagnetic field, the susceptor is heated. In general, this may be the result of hysteresis losses and/or eddy currents induced in the susceptor, depending on the electrical and magnetic properties of the susceptor material. Hysteresis losses occur in ferromagnetic or ferrimagnetic susceptor materials due to magnetic domains within the material being switched under the influence of an alternating electromagnetic field. Eddy currents may be induced if the susceptor material is electrically conductive. In case of an electrically conductive ferromagnetic or ferrimagnetic susceptor material, heat can be generated due to both, eddy currents and hysteresis losses. Accordingly, the open-porous inductively heatable ceramic material according to the invention may be heatable due to at least one of hysteresis losses or eddy currents, depending on the electrical and magnetic properties of the open-porous ceramic material. Accordingly, the open-porous inductively heatable ceramic material may be electrically conductive. Alternatively or additionally, the open-porous inductively heatable ceramic material may be ferromagnetic or ferrimagnetic. For example, the susceptor may comprise or consist of an electrically conductive ceramic material, such as lanthanum-doped strontium titanate, or yttrium-doped strontium titanate. Likewise, the susceptor may comprise or consist of an open-porous ferrimagnetic or ferromagnetic ceramic material, such as a ceramic ferrite.

As used herein, the term 'aerosol-forming liquid' relates to a liquid capable of releasing volatile compounds that can form an aerosol upon heating the aerosol-forming liquid. The aerosol-forming liquid may contain both, solid and liquid aerosol-forming material or components. The aerosol-forming liquid may comprise a tobacco-containing material containing volatile tobacco flavour compounds, which are released from the liquid upon heating. Alternatively or additionally, the aerosol-forming substrate may comprise a non-tobacco material. The aerosol-forming liquid may further comprise an aerosol former. Examples of suitable aerosol formers are glycerine and propylene glycol. The aerosol-forming substrate may also comprise other additives and ingredients, such as nicotine or flavourants. In particular, the aerosol-forming liquid may include water, solvents, ethanol, plant extracts and natural or artificial flavours. The aerosol-forming liquid may also be a paste-like material, a sachet of porous material comprising aerosol-forming substrate, or, for example, loose tobacco mixed with a gelling agent or sticky agent, which could include a common aerosol former such as glycerine, and then is compressed or molded into a plug.

The specific material and geometry of the susceptor can be chosen to provide a desired heat generation and liquid absorption and retention effect. In general, the susceptor may have any desired shape. The shape may depend from the specific place of action and installation when used with an aerosol-generating article, device or system. For example, the susceptor may be of one of a cylinder, a disc, a tube, a cuboid or a washer-shaped configuration.

The susceptor may be a unitary body comprising or being made of the open-porous inductively heatable ceramic material. The unitary body may be a compact solid body. This configuration advantageously allows for providing a compact unitary storage medium for aerosol-forming liquid to be heated. In particular, the unitary susceptor body may be a unitary pellet or pressed article.

Alternatively, the susceptor may comprise a plurality of susceptor elements, wherein each susceptor element comprises or is made of or consists of the open-porous inductively heatable ceramic material. Likewise, each susceptor element may be a unitary body, in particular a compact solid body. For example, the susceptor may be a solid bulk material of individual susceptor elements, such as individual susceptor pellets. The susceptor may be a susceptor granulate.

The quantity of aerosol-forming liquid which is held and heated by the susceptor is a related to porosity of the open-porous ceramic material. Preferably, the open-porous inductively heatable ceramic material has a porosity between 20 % and 60%. A porosity in this range proves advantageous with regard to the amount of aerosol-forming liquid that is held and heated by the susceptor in order to provide a convenient user experience. The porosity may be chosen such that the susceptor holds a predetermined amount of aerosol-forming liquid. The predetermined amount of liquid preferably corresponds to predefined number of puffs to be available when using the susceptor with an aerosol-generating device or system. The porosity may also be chosen with regard to a specific air-flow management through the susceptor. For example, the porosity may be chosen such as to provide a specific resistance-to-draw (RTD).

Preferably, heating of the aerosol-forming liquid is based on hysteresis losses only. Therefore, heating of the susceptor, that is, heating of the open-porous inductively heatable ceramic material, mainly or even exclusively may result from hysteresis losses. Therefore, the open-porous ceramic material preferably is ferrimagnetic or ferromagnetic only. Accordingly, the open-porous inductively heatable ceramic material preferably is electrically non-conductive or - if at all - only a very weakly conductive. As will be described in more detail below, this may be desirable in order to limit the heatability of the susceptor to a temperature corresponding to the Curie temperature of the susceptor material. In an electrically non-conductive material, eddy currents and thus heating due to eddy currents do not occur.

Ferrimagnetic materials and ferromagnetic materials are characterized in that they hold a spontaneous magnetization below the Curie temperature, and show no magnetic order above this temperature. Therefore, above its Curie temperature ferrimagnetic or ferromagnetic materials are paramagnetic and so heating due to hysteresis losses no longer occurs. Thus, in case the open-porous ceramic material of the susceptor is electrically non-conductive, but ferrimagnetic or ferromagnetic only, the inductive heatability even completely disappears above the Curie temperature. This effect may be advantageously used for controlling the heating temperature of the susceptor. Accordingly, the open-porous inductively heatable ceramic material of the susceptor may have a Curie temperature chosen such as to correspond to a maximum temperature to which the susceptor should be heated in order to avoid or at least reduce the possibility of rapid overheating. The Curie temperature may deviate from this maximum temperature by about 1% to 3%. The inductively heatable ceramic material of the susceptor may be selected to have a Curie temperature lower than 400 °C, preferably lower than 380 °C, or lower than 360 °C. Preferably, the inductively heatable ceramic material has a Curie temperature between 150 °C and 300 °C. This holds in particular for those susceptors comprising only one single ferrimagnetic ceramic material.

As mentioned above, the open-porous inductively heatable ceramic material preferably is a ceramic ferrite. As used herein, ferrites are ferrimagnetic ceramic compounds derived from iron oxides such as hematite (Fe₂O₃) or magnetite (Fe₃O₄) as well as oxides of other metals. Usually, ferrites are electrically non-conductive.

In particular, the open-porous inductively heatable ceramic material may comprise or may be at least one of:
- a manganese-magnesium ferrite;
- a nickel-zinc ferrite; or
- a cobalt-zinc barium ferrite.

The nickel-zinc ferrite - as mentioned above - may comprise or may consist of a composition of the type Mgₓ Mn_{y} Fe_{z} O₄, wherein x = 0.4 - 1.1, y = 0.3 - 0.9, and z = 1 - 2, and wherein the atomic fraction x, y and z of the metallic cations Mg, Mn and Fe is such that the total charge of the metallic cations equilibrates the total charge of the oxygen anions. In particular, the open-porous inductively heatable ceramic material may comprise or may be one of:
- Mg_{0.77} Mn_{0.58} Fe_{1.65} O₄, having a Curie temperature of about 270 °C;
- Mg_{0.55} Mn_{0.88} Fe_{1.55} O₄; having a Curie temperature of about 262 °C;
- Mg_{1.03} Mn_{0.35} Fe_{1.37} O₄; having a Curie temperature of about 190 °C.

The nickel-zinc ferrite - as mentioned above - may comprise or may consist of a composition of the type Niₓ Zn₁₋ₓ Fe₂ O₄, wherein x = 0.3 - 0.7 and the atomic fraction of the metallic cations Ni, Zn and Fe is such that the total charge of the metallic cations equilibrates the total charge of the oxygen anions. In particular, the open-porous inductively heatable ceramic material may comprise or may be for example Ni_{0.5} Zn_{0.5} Fe₂ O₄, having a Curie temperature of about 258 °C.

The cobalt-zinc barium ferrite - as mentioned above - may comprise or may consist of Co_{1.75} Zn_{0.25} Ba2 Fe₁₂ O₂₂, having a Curie temperature of about 279 °C.

A method for producing a susceptor comprising an open-porous inductively heatable ceramic material according to the invention may comprise the following steps:
- mixing powdered raw components of the ceramic material;
- dissolving cellulose into a solvent;
- mixing the dissolved cellulose with the mixed raw components to get a slurry mixture;
- drying the slurry mixture;
- pressing the dried mixture to form a pellet of desired shape;
- calcinating the pellet to form an open-porous pellet;
- annealing the open-porous pellet.

The steps of mixing the powdered raw components of the ceramic material and mixing the dissolved cellulose with the mixed raw components may be combined, that is the raw components of the ceramic material and the dissolved cellulose may be mixed together in a single step.

Instead of using a solvent, processing of the cellulose and the powdered raw materials may alternatively be done in dry condition. Therefore, an alternative method for producing a susceptor comprising an open-porous inductively heatable ceramic material according to the invention may comprise the following steps:
- mixing powdered raw components of the ceramic material and cellulose to get a dry mixture;
- pressing the dry mixture to form a pellet of desired shape;
- calcinating the pellet to form an open-porous pellet;
- annealing the open-porous pellet.

As used herein, 'calcinating' is a thermal treatment process in an air or oxygen atmosphere at a temperature between 550 °C and 1300 °C. Calcination may be performed in a calciner. A calciner may be a steel cylinder that rotates inside a heated furnace and performs indirect high-temperature processing within a controlled atmosphere. With regard to the ceramic material according to the invention, calcination aims to combust the cellulose and - if present - to remove the solvent. During this process, the desired open-porous structure of the ceramic material is formed. Preferably, the pellet is calcinated at a temperature of about 1200 °C.

The cellulose has two functions. First, the cellulose acts as binder between the particles of the mixed raw components in the pellet. Second, the cellulose particles advantageously act as displacement bodies to form the open-porous structure.

The pressure applied to the dried mixture to form a pellet of desired shape may be in a range of 5-10 t/cm² (tons per square centimetre). For example, a load of 10 tons may be applied to a circular sample having a diameter 13 mm.

The open-porous pellet is preferably annealed at a temperature in the range of 500 °C to 700°C, in particular at a temperature of about 600 °C.

Prior to the step of the mixing the powdered raw components, the method may further comprise the step of sieving the raw components of the ceramic material to select powder particles of the raw components having a specific grain size in a desired range. Preferably, the specific grain size is between 50 µm and 80 µm.

The method may further comprise the step of milling the raw components prior to the mixing the raw components, and - if provided - prior to sieving the raw components.

After the step of milling, the method may further comprise the step of drying the milled raw components prior to the mixing the raw components, and - if provided - prior to sieving the raw components.

Preferably, the susceptor may be part of or may be a consumable aerosol-generating article which is pre-soaked with an aerosol-forming liquid such as to be ready for use with an aerosol-generating device including an induction source. Therefore, the susceptor may further comprise an aerosol-forming liquid held in the open-porous inductively heatable ceramic material. That is, the susceptor may comprise an open-porous inductively heatable ceramic material which holds an aerosol-forming liquid or which is (pre-)soaked with an aerosol-forming liquid. In particular, the open-porous inductively heatable ceramic material may hold or may be (pre-) soaked with a predetermined amount of an aerosol-forming liquid. The predetermined amount of liquid preferably corresponds to predefined number of puffs to be available when using the susceptor with an aerosol-generating device.

Alternatively, the susceptor may be integral part of an aerosol-generating device. Therefore, the invention also provides an aerosol-generating device for generating an aerosol by inductively heating an aerosol-forming liquid. The aerosol-generating device comprises an induction source comprising an induction coil for generating an alternating electromagnetic field. Furthermore, the device comprises a susceptor according to invention and as described herein which includes an open-porous inductively heatable ceramic material for holding and heating the aerosol-forming liquid. The susceptor is positioned relative to the induction coil so as to be inductively heatable by the alternating electromagnetic field in operation of the device.

For generating the alternating electromagnetic field, the induction source may comprise an alternating current (AC) generator. The AC generator may be powered by a power supply of the aerosol-generating device. The AC generator is operatively coupled to the induction coil. The AC generator is configured to generate a high frequency oscillating current to be passed through the induction coil for generating an alternating electromagnetic field. As used herein, a high frequency oscillating current means an oscillating current having a frequency between 500 kHz and 30 MHz, preferably between 1 MHz and 10 MHz and more preferably between 5 MHz and 7 MHz.

The device may further comprise an electric circuitry which preferably includes the AC generator. The electric circuitry may advantageously comprise a DC/AC inverter, which may include a Class-D or Class-E power amplifier. The electric circuitry may be connected to an electrical power supply of the aerosol-generating device. The electric circuitry may comprise a microprocessor, which may be a programmable microprocessor, a microcontroller, or an application specific integrated chip (ASIC) or other electronic circuitry capable of providing control. The electric circuitry may comprise further electronic components. The electric circuitry may be configured to regulate a supply of current to the induction coil. Current may be supplied to the induction coil continuously following activation of the system or may be supplied intermittently, such as on a puff by puff basis.

As already mentioned above, the aerosol-generating device advantageously comprises a power supply, preferably a battery such as a lithium iron phosphate battery. As an alternative, the power supply may be another form of charge storage device such as a capacitor. The power supply may require recharging and may have a capacity that allows for the storage of enough energy for one or more user experiences. For example, the power supply may have sufficient capacity to allow for the continuous generation of aerosol for a period of around six minutes or for a period that is a multiple of six minutes. In another example, the power supply may have sufficient capacity to allow for a predetermined number of puffs or discrete activations of the induction coil.

The device may comprise a single induction coil or a plurality of induction coils. The number of induction coils may depend on the number of susceptor elements. The induction coil or coils may have a shape matching the shape of the susceptor. Likewise, the induction coil or coils may have a shape to conform to a shape of a housing of the aerosol-generating device. For example, the induction coil or coils may be a helical coil or flat spiral coil. The induction coil may be wound around a ferrite core. As used herein a 'flat spiral coil' means a coil that is generally planar coil wherein the axis of winding of the coil is normal to the surface in which the coil lies. The flat spiral induction can have any desired shape within the plane of the coil. For example, the flat spiral coil may have a circular shape or may have a generally oblong or rectangular shape. However, the term 'flat spiral coil' as used herein covers coils that are planar as well as flat spiral coils that are shaped to conform to a curved surface. The use of a flat spiral coil allows for designing a compact device, having a simple design that is robust and inexpensive to manufacture. The coil can be held within a housing of the device and need not to be exposed to generated aerosol so that deposits on the coil and possible corrosion can be prevented. The induction coil may be covered by a corrosion resistant coating or enclosure. The induction coil may have a diameter of between 5 mm and 10 mm. The induction coil may be positioned on or adjacent a surface of cavity closest to the power supply. This reduces the amount and complexity of electrical connections within the device.

In use, it is advantageous to have the susceptor close to the induction coil such as to ensure that the alternating electromagnetic field permeates the open-porous inductively heatable ceramic material. Advantageously, the susceptor is positioned in the vicinity of the induction coil. It is also desirable that the distance between the induction coil and the susceptor is substantially constant across the extent of the susceptor as to ensure homogenous heating. Preferably, the minimum distance between the susceptor and the induction coil is below 2 mm, in particular below 1 mm, or even below 0.5 mm.

The aerosol-generating device may comprise a device housing. The device housing may comprise the susceptor, the induction source, the induction coil, the AC generator, the electric circuitry, and the power supply. As will be further described below, the device housing may further comprise a tank or a liquid retention element, or both, for storing aerosol-forming liquid.

The device housing may further comprise a cavity in which the susceptor may be at least partially arranged. The cavity may have an internal surface. The induction coil may be positioned on or adjacent a surface of the cavity closest to the power supply. The induction coil may be shaped to conform to the internal surface of the cavity. Alternatively, the induction coil may be within the cavity. In particular, the cavity may be an aerosol-generating chamber.

The device housing may comprise a main body and a mouthpiece portion. The cavity may be in the main body and the mouthpiece portion may have an outlet through which aerosol generated by the device can be drawn out. The induction coil may be arranged in the main body, in the mouthpiece portion, or in both, the main body and the mouthpiece portion. As used herein, the term 'mouthpiece portion' means a portion of the device that is placed into a user's mouth in order to directly inhale an aerosol generated by the aerosol-generating system. The aerosol is conveyed to the user's mouth through the mouthpiece.

The device may comprise an air path extending from at least one air inlet to at least one air outlet. The air outlet preferably is an outlet of a mouthpiece. The air path passes the susceptor, in particular an external surface of the open-porous ceramic material. The air path may go through the cavity. The air path may also pass the induction coil. By allowing the air flow through the device to pass through the coil a compact system can be achieved. The induction coil may be positioned adjacent to the susceptor. The air path may include an airflow passage provided between the induction coil and the susceptor element. Vaporized aerosol-forming material may be entrained in the air flowing in the airflow passage, which subsequently cools to form an aerosol that escapes through the air outlet.

The open-porous ceramic material of the susceptor may be (pre-)soaked with a predetermined amount of an aerosol-forming liquid, for example for a single use of the device. Yet, multiple use of the device and the susceptor integrated therein may be preferred. Therefore, the device may be configured for repeatedly or continuously soaking the susceptor with aerosol-forming liquid. For this, the aerosol-generating device may further comprise a tank for holding or storing aerosol-forming liquid. The tank may be replaceable or refillable. The tank may be arranged within a housing of the device, in particular within the main body of the device. For (resoaking) the susceptor with aerosol-forming liquid the tank is in fluid communication with the susceptor, for example via a fluid channel or a fluid pipe.

Transfer of aerosol-forming liquid from the tank to the susceptor preferably occurs due to gravity. Alternatively, the liquid transfer may occur due to capillary effects, such as via a capillary wick element between the tank and the susceptor. The aerosol-generating device may also comprise a pumping device, such as a micro-pump, for transferring aerosol-forming liquid from the tank to the susceptor.

Preferably, the aerosol-generating device may be configured such that soaking of the susceptor with aerosol-forming liquid from the tank only occurs in a specific position of the device, for example an up-down or an overhead position of the device. As used herein, position of the device primarily refers to the orientation of the device in space, in particular with regard to gravity. In other words, the aerosol-generating device may be configured such that soaking of the susceptor with aerosol-forming liquid from the tank requires orientating the device into a specific position. The specific position may be denoted as 'soaking position'. Advantageously, this reduces the risk of unwanted soaking or even oversoaking of the susceptor beyond its capacity.

The aerosol-generating device may be configured such that transfer of aerosol-forming liquid from the tank to the susceptor preferably occurs due to gravity only. For this, the relative arrangement between the susceptor and the tank may be such that in a specific soaking position of the device, the susceptor is arranged at a level below a level of the tank. In contrast, in an operation position of the device, that is, during aerosol generation, the susceptor is preferably arranged at a level above a level of the tank. Accordingly, there is no transfer of aerosol-forming liquid from the tank to the susceptor in the operation position. If at all, excess aerosol-forming liquid may reflow in the operation position from the susceptor or the fluid channel/fluid pipe into the tank.

Alternatively or in addition, the fluid communication between the susceptor and the tank is interruptible or releasable. In particular, the aerosol-generating device may be configured such that the tank is in fluid communication with the susceptor only in a specific soaking position of the device. At least in the operation position of the device, but also in any position other than the soaking position, the fluid communication may be disabled, released, interrupted or shut-off. For realizing an interruptible or releasable fluid communication, the aerosol-generating device may comprise a valve for controlling the fluid communication between the tank and the susceptor. The valve may be a gravity-actuated valve that is open only in a specific position of the device, such as an up-down or an overhead position of the device. The valve may be a controllable electromagnetic valve. The electromagnetic valve may be manually controllable, for example by a switch. Alternatively, the electromagnetic valve may be coupled to an electric circuitry of the aerosol-generating device for controlling the shutting-off and opening of the valve. The electric circuitry may further comprise a position sensor, such as a microchip-packaged MEMS gyroscope, for determining the position of the aerosol-generating device. Accordingly, the electric circuitry may be configured to open the electromagnetic valve only in case the position sensor detects that the aerosol-generating device is in a specific position. In case the position sensor detects any other position, the valve is closed by the electric circuitry.

The aerosol-generating device may be further configured such that heating of the susceptor is disabled during soaking of the susceptor with aerosol-forming liquid. Advantageously, this prevents unintentional gas formation in the tank.

The aerosol-generating device may be configured such that an aerosol passage towards an aerosol output of the aerosol-generating device is closed during soaking of the susceptor with aerosol-forming liquid. Advantageously, this prevents unintentional absorption of aerosol-forming liquid by a user of the device.

Due to the open-porous structure of the ceramic material, the susceptor already provides high liquid retention capacity. Nevertheless, the aerosol-generating device may further comprise a liquid retention element for holding additional aerosol-forming liquid. The liquid retention element may comprise a high retention or high release material (HRM) for storing liquid aerosol-forming substrate. Advantageously, the liquid retention element may be a storage medium for aerosol-forming liquid to soak the susceptor with. For this, the liquid retention element is preferably in direct contact with the susceptor. Thus, aerosol-forming liquid stored in the liquid retention element may be easily transferred to the susceptor, for example by capillary action. Aerosol-forming liquid retained in the liquid retention element preferably is not available for aerosolization before having left the retention element. The liquid retention element may be electrically non-conductive. The liquid retention element may also be paramagnetic or diamagnetic. Preferably, the liquid retention element may be inductively non-heatable. The liquid retention element may be arranged with the aerosol-generating device such as to be unaffected or only minimally affected by the alternating electromagnetic field of the induction coil.

The aerosol-generating device may comprise both, a liquid retention element and a tank for aerosol-forming liquid. Preferably, the tank is in fluid communication with the liquid retention element, which in turn may be in fluid communication with the susceptor. Thus, the liquid retention element is (re-)filled from the tank, whereas the susceptor is soaked from the liquid retention element.

As mentioned above, the susceptor may be part of or may be a consumable aerosol-generating article which is pre-soaked with an aerosol-forming liquid such as to be ready for use with an aerosol-generating device that includes an induction source. Preferably, the aerosol-generating article may be part of or may be a cartridge for use with an aerosol-generating device. Therefore, the invention also provides a cartridge for use with an inductively heated aerosol-generating device. The cartridge comprises an aerosol-forming liquid and an inductively heatable susceptor according to the invention and as described herein. The susceptor comprises or is made of or consists of an open-porous inductively heatable ceramic material as described herein which holds at least a portion of the aerosol-forming liquid contained in the cartridge. In addition to holding at least a portion of the aerosol-forming liquid, the inductively heatable ceramic material allows for inductively heating the aerosol-forming liquid held therein under the influence of an alternating electromagnetic field

The cartridge is a consumable, in particular disposable aerosol-generating article. It is configured to be received in a cavity of an aerosol-generating device which in turn comprises an induction source for inductively heating the susceptor of the cartridge when it is received in the cavity. In operation, the induction source generates an alternating magnetic field that permeates the susceptor of the cartridge received in the cavity. Depending on the electrical and magnetic properties of the inductively heatable ceramic material, the alternating magnetic field causes at least one of eddy currents or hysteresis losses in the susceptor. Consequently, the susceptor heats up causing the aerosol-forming liquid held therein to be vaporized. Due to the open-porous structure of the ceramic material, the vaporized aerosol-forming liquid can pass through the susceptor and subsequently cool to form an aerosol.

Preferably, the susceptor holding the aerosol-forming liquid may essentially constitute the cartridge, that is, the consumable aerosol-generating article. In this case, the susceptor may hold the entire aerosol-forming liquid of the cartridge. In other words, the cartridge according to the invention may only consist of the susceptor soaked with aerosol-forming liquid. Advantageously, such a cartridge proves to be simple, inexpensive and robust.

In addition, the cartridge may comprise a cartridge housing surrounding the soaked susceptor at least partially. Preferably, the cartridge housing surrounds the susceptor completely, that is, the susceptor may be within the cartridge housing.

When the cartridge housing is to be received in a cavity of an aerosol-generating device, the housing preferably is electrically non-conductive.

The susceptor may fill at least a portion of an interior space of the cartridge housing.

The cartridge housing may be at least partially or completely removable such as to at least partially or completely free the susceptor. In operation, this allows the vaporized aerosol-forming liquid to freely escape from the cartridge, and vice versa, to let air enter into the susceptor. In particular in case the cartridge constitutes a consumable aerosol-generating article essentially consisting of the susceptor soaked with aerosol-forming liquid, the cartridge housing may be an envelope or a cover of the susceptor which can be at least partially or completely removed prior to engaging the cartridge with an aerosol-generating device, that is prior to engaging the partially or completely freed susceptor with an aerosol-generating device.

The cartridge housing may comprise at least one fluid permeable portion. As used herein a 'fluid permeable portion' is a portion of the cartridge housing allowing gas, preferably also liquid to permeate through it. In particular, the at least one fluid permeable portion of the cartridge housing allows the aerosol-forming liquid, in either gaseous phase or both gaseous and liquid phase, to permeate through it. The cartridge housing may have a plurality of fluid permeable portions. Preferably, at least a part of those portions of the cartridge housing covering the susceptor or being in contact with the susceptor may be fluid permeable. Even the entire cartridge housing may be fluid permeable. The latter configuration proves advantageous with regard to a cartridge that is completely filled by a susceptor soaked with aerosol-forming liquid or with regard to a cartridge which essentially consists of a susceptor soaked with aerosol-forming liquid.

Due to the high retention capacity of the susceptor material, the susceptor itself may also form at least a portion of the cartridge housing. The susceptor may even form the complete cartridge housing. As an example, the cartridge may be a hollow cylinder comprising a circumferential wall and two end walls. The circumferential wall and the end walls form a housing of the cartridge. At least one end wall or at least a portion of the circumferential wall, or both, may be formed by the susceptor.

The susceptor may only partially fill the volume of the cartridge housing. Advantageously, the void interior volume of the cartridge may be used as tank or reservoir filled with aerosol-forming liquid. A portion of the surface of the susceptor facing the interior of the cartridge may be in direct contact with the aerosol-forming liquid. Thus, when heated aerosol-forming liquid held in the susceptor is vaporized and released from the cartridge through the open-porous structure of the ceramic susceptor material. At the same time, the susceptor is continuously re-filled or resoaked by aerosol-forming liquid stored in the cartridge tank or reservoir. As compared to a cartridge in which the susceptor completely fills the cartridge volume, a cartridge having a void volume filled with aerosol-forming liquid has a larger operation time. This is due to the fact that the liquid storage capacity of the susceptor volume is lower as compared to a free volume of equal size.

The overall surface corresponding to the outer contour of the susceptor body present on the outer surface of the cartridge may amount about 25 mm².

Further advantages and features of the cartridge according to the invention have been described above with regard to the susceptor and will not be repeated.

According to the invention there is also provided an aerosol-generating system for generating an aerosol by inductively heating an aerosol-forming liquid. The system comprises an aerosol-generating device and a cartridge according to the invention and as described herein. Accordingly, the cartridge comprises an aerosol-forming liquid and an inductively heatable susceptor according to invention and as described herein which holds at least a portion of the aerosol-forming liquid. The cartridge is configured to be used with the aerosol-generating device, that is, to be engaged with the aerosol-generating device for generating an aerosol by inductively heating the aerosol-forming liquid contained in the cartridge. For this, the aerosol-generating device comprises a device housing including a cavity for receiving at least a portion of the cartridge. The aerosol-generating device further comprises an induction source within the device housing comprising an induction coil for generating an alternating electromagnetic field. The aerosol-generating device and the cartridge are configured such that upon receiving the cartridge in the cavity the susceptor is positioned relative to the induction coil so as to be inductively heatable by the alternating electromagnetic field.

The induction coil may be positioned on or adjacent an internal surface of the cavity. The induction coil may be shaped to conform to the internal surface of the cavity. Alternatively, the induction coil may be within the cavity. In some embodiments, the induction coil may be within an internal passage of the cartridge when the cartridge is engaged with the device.

The device housing may comprise a main body and a mouthpiece portion. The cavity may be in the main body and the mouthpiece portion may have an outlet through which aerosol generated by the system can be drawn out. The induction coil may be in the mouthpiece portion or in the main body. Alternatively a mouthpiece portion may be provided as part of the cartridge.

The device may comprise an air path extending from at least one air inlet to at least one air outlet. The air outlet preferably is an outlet of a mouthpiece. The air path passes the susceptor, in particular an external surface of the open-porous ceramic material. The air path may go through the cavity. The air path may also pass the induction coil. By allowing the air flow through the device to pass through the coil a compact system can be achieved. In use, the induction coil may be positioned adjacent to the susceptor when the cartridge is engaged with the device, that is, received in the cavity. The air path may include an airflow passage provided between the induction coil and the susceptor element when the cartridge is received in the cavity. Vaporized aerosol-forming material may be entrained in the air flowing in the airflow passage, which subsequently cools to form an aerosol and may be escape through the air outlet.

As compared to the aerosol-generating device described above, the aerosol-generating device described here does neither comprise an internal susceptor nor an internal reservoir for aerosol-forming liquid, such as a liquid tank. However, apart from that, the aerosol-generating device described here may be similar or identical to the aerosol-generating device described above.

In particular, the induction source and the induction coil of the aerosol-generating device described here may be similar or identical to the induction source and the induction coil of the aerosol-generating device described above. Likewise, the aerosol-generating device described here may also comprise at least one of an AC generator, an electric circuitry, and a power supply as described above.

Further features and advantages of the aerosol-generating device described here, in particular further features and advantages of the induction source, the induction coil, the AC generator, the electric circuitry, and the power supply have been described with regard to the aerosol-generating device described above and will not be repeated.

The invention will be further described, by way of example only, with reference to the accompanying drawings, in which:
- Fig. 1: schematically illustrates an aerosol-generating system in accordance with a first embodiment of the invention;
- Fig. 2: shows a sectional view of the aerosol-generating system according to Fig. 1 along line A-A;
- Fig. 3: schematically illustrates an aerosol-generating system in accordance with a second embodiment of the invention;
- Fig. 4: schematically illustrates a first embodiment of a cartridge in accordance with the invention;
- Fig. 5: shows a perspective view of the cartridge according to Fig. 4;
- Fig. 6: schematically illustrates a second embodiment of a cartridge in accordance with the invention;
- Fig. 7: shows a perspective view of the cartridge according to Fig. 6;
- Figs. 8-17: schematically illustrate further embodiments of the cartridge according to the invention;
- Fig. 18: schematically illustrates an aerosol-generating device in accordance with a first embodiment of the invention; and
- Fig. 19: schematically illustrates an aerosol-generating device in accordance with a second embodiment of the invention.

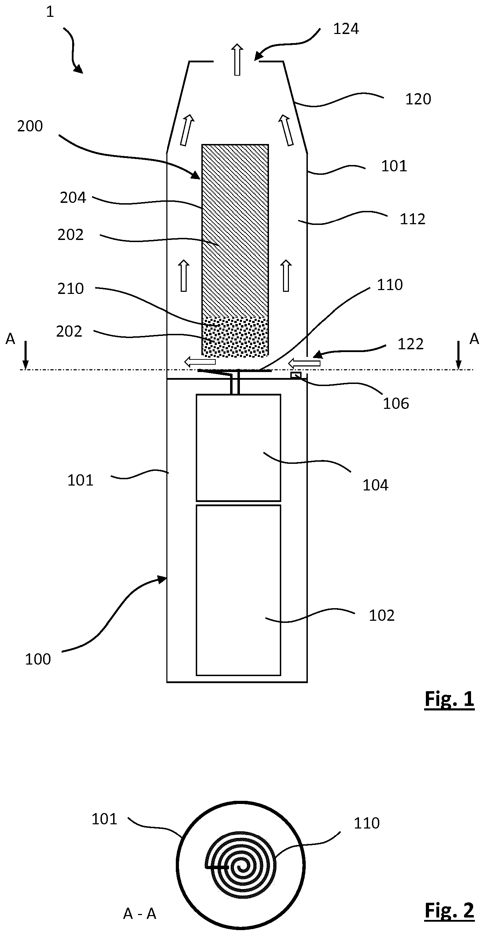

**Fig. 1** is a schematic illustration of an aerosol-generating system 1 in accordance with a first embodiment of the invention. The system comprises an aerosol-generating device 100 and a cartridge 200 to engage with the aerosol-generating device 100. The device 100 comprises a main body having a main body housing 101 which contains a lithium ion battery as power supply 102, and a control electric circuitry 104. The main body housing 101 defines a cavity 112 in which the cartridge 200 is received. The device 100 also includes a mouthpiece portion 120 comprising an outlet 124. A housing of the mouthpiece portion 120 and the main body housing 101 together form the housing of the device 100. The mouthpiece portion may be connected to the main body by any kind of connection, such as by a hinged connection, a snap fitting or a screw fitting. Air inlets 122 are defined in the main body housing 101.

A flat spiral induction coil 110 is arranged within the cavity 112. The coil 110 is operatively connected to the control electric circuitry 104. The coil 110 is also illustrated in **Fig. 2****.** The coil 110 is formed by stamping or cutting a spiral coil from a sheet of copper. The coil 110 is positioned close to an inner surface of the cavity 112, opposite to an end surface of the cartridge 200, at the level of the air inlets 122. Thus, air drawn through the inlets 122 towards to the outlet 124 passes through a passage way formed between the coil 112 and the end surface of the cylindrical cartridge 200. Advantageously, a flat spiral coil allows for a simple interface between the device and the cartridge, which in turn allows for a simple and inexpensive cartridge design.

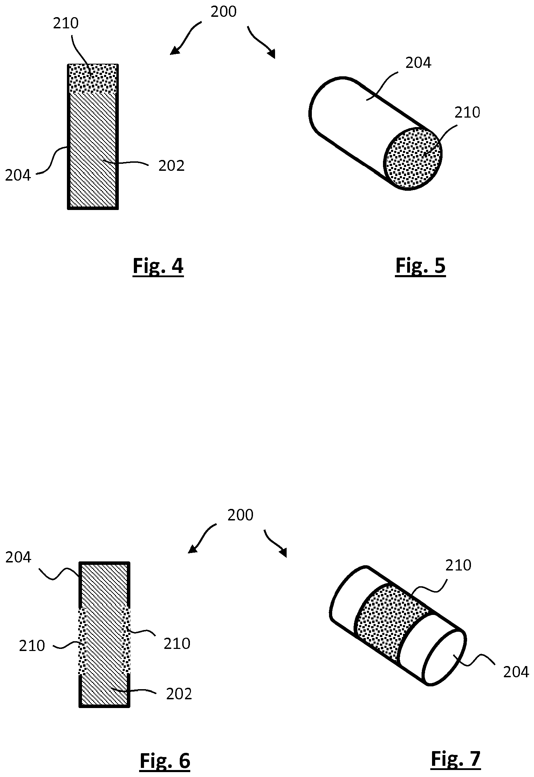

In this embodiment, the cartridge 200 is of circular cylindrical shape. The cylindrical cartridge 200 comprises a cartridge housing 204 containing an aerosol-forming liquid 202. The aerosol-forming liquid may be held by a capillary material. The cartridge housing 204 is fluid impermeable but has an open end covered by a susceptor 210. Further details of the cartridge 200 are illustrated in **Fig. 4** and **Fig. 5****.** In this embodiment, the susceptor 210 is a compact solid susceptor body made of an open-porous ferrimagnetic ceramic material, for example Ni_{0.5} Zn_{0.5} Fe2 O₄. The susceptor body 210 is of cylindrical shape and inserted into the open end of the cartridge housing 204. Thus, the susceptor 210 forms at least a portion of the cartridge housing 204. The cylindrical susceptor body 210 has an axial length between 3 mm and 6 mm, preferably between 4 and 5 mm. The overall surface corresponding to the cylindrical outer contour of the susceptor body 21 may amount about 25 mm².

An inner end surface of the cylindrical susceptor body 210 faces the interior of the cartridge housing 204 such as to be in direct contact with the aerosol-forming liquid 202 contained in the cartridge 200. Due to the open-porous structure of the ceramic material, the susceptor is soaked with aerosol-forming liquid 202. Accordingly, the susceptor 210 holds a least of portion of the aerosol-forming liquid 202 contained in the cartridge 200. An outer end surface of the cylindrical susceptor body 210 forms an outer surface of the cartridge 200. Thus, when heated aerosol-forming liquid held in the susceptor is vaporized and may freely escape from the cartridge 200 via the outer end surface of the open-porous susceptor body 210.

The open-porous structure is such as to provide a high retention capacity for liquid aerosol-forming material. Due to this, aerosol-forming liquid is safely held or retained in the susceptor 210. Advantageously, this allows the cartridge 200 to be leak proof with regard to the aerosol-forming liquid 202 contained therein, even though a portion of the cartridge housing is made of an open-porous material. Vice versa, the open porosity of the susceptor material is such as to allow vaporized aerosol-forming material to be freely released from the cartridge upon heating.

When the cartridge 200 is engaged with the aerosol-generating device 100 and received in the cavity 112, the susceptor element 210 is positioned adjacent the flat spiral coil 110. The cartridge 200 may include keying features to ensure that it cannot be inserted into the device upside-down.

In use, a user may puff on the mouthpiece portion 120 to draw air though the air inlets 122 into the cavity 112 and the mouthpiece portion 120 and out of the outlet 124 into the user's mouth. The device may include a puff sensor 106 in the form of a microphone for detecting when a user puffs on the mouthpiece. The puff sensor 106 may be part of the control electric circuitry 104. The puff sensor 106 may be arranged within the cavity close to the air inlets 122. When a puff is detected, the electric circuitry 104 provides a high frequency oscillating current to the coil 110. This generates an oscillating magnetic field which passes through the susceptor 210. As a consequence, the susceptor 210 heats up due to hysteresis losses and reaches a temperature sufficient to vaporize the aerosol-forming liquid held in the open pores of the susceptor material. The vaporized aerosol-forming material is entrained in the air flowing from the air inlets 122 towards the air outlet 124. Along this way, the vapor cools to form an aerosol within the mouthpiece portion 120 before escaping through the outlet 124. The control electric circuitry 104 supplies the oscillating current to the coil 110 for a predetermined duration, in this example five seconds, after detection of a puff and then switches the current off until a new puff is detected.

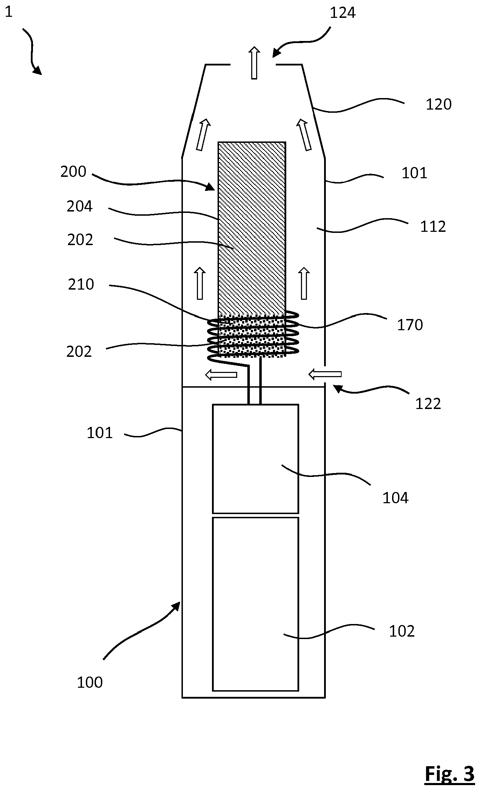

**Fig. 3** is a schematic illustration of an aerosol-generating system 1 in accordance with a second embodiment of the invention. The system 1 comprises an aerosol-generating device 100 and a cartridge 200. Except for the induction coil, this second embodiment is identical to the first embodiment shown in Fig. 1. Therefore, in both embodiments identical features of the aerosol-generating device and the cartridge are denoted with identical reference numerals. Instead of a flat spiral induction coil, the aerosol-generating device 100 according to the second embodiment comprises a helical induction coil 170 positioned in the cavity 112 so that the susceptor 210 is arranged within the helical induction 170 when the cartridge is received in the cavity 112. Applying a high frequency oscillating current to the coil 170 generates an oscillating magnetic field which is essentially homogenous in the interior of the helical coil 170. Thus, when the cartridge 200 is engaged with the aerosol-generating device 100, the susceptor 120 is homogeneously affected by the oscillating magnetic field which proves advantageous with regard to a homogenous heating of the susceptor. Alternatively, the helical induction 170 may be also arranged on the inner surface of the cavity 112 or even within the wall of the main body housing 101, which allows for a simple and compact design of the aerosol-generating device 100.

The cartridge 200 according to the embodiment shown in Figs. 1 to 5 has a simple and robust design, which can be inexpensively manufactured as compared to the cartomizers available on the market. However, other configurations are possible as shown in Figs. 6 to 16.

**Fig. 6** and **Fig. 7** schematically show an alternative cartridge design having a hollow cylindrical shape. The susceptor 210 is a compact susceptor body made of an open-porous ferrimagnetic ceramic material which forms a circumferential portion of a circumferential wall of the cartridge housing 204. Thus, the susceptor 220 is of tubular shape. The interior of the hollow cartridge 200 contains aerosol-forming liquid, a portion of which is held in the susceptor 210. The cartridge as shown in Figs. 6 and 7 may be designed to engage with an aerosol-generating device comprising a helical induction coil as shown in Fig. 3. The aerosol-generating device is preferably designed such that the susceptor is coaxially positioned within the interior of the helical coil when the cartridge is engaged with the aerosol-generating device. Thus, heating of the susceptor advantageously is very homogenous. Furthermore, the active heating volume of the cartridge design shown in Figs. 6 and 7 is larger as compared to the cartridge design shown in Figs. 4 and 5, leading to a more intensive user experience.

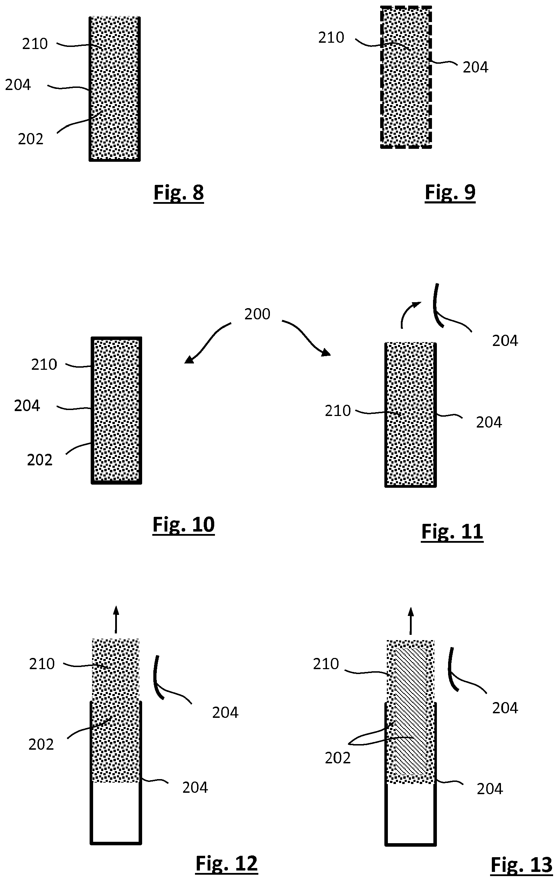

**Fig. 8** schematically shows a sectional view of another cartridge design in which the cartridge is completely filled by a susceptor 210. In this embodiment, the susceptor 210 is a compact susceptor body made of an open-porous ferrimagnetic ceramic material providing high retention for liquid aerosol-forming substrate. Therefore, the cartridge 200 of the present embodiment advantageously reduces the risk of spill, for example as compared to a liquid tank. In case of cracks of failures of the cartridge, the high retention material of the susceptor avoids unintended contact of aerosol-forming liquid with active electrical components of the device and biological tissue.

The cartridge 200 comprises a cartridge housing 204 which at least partially surrounds the susceptor 210. In use, vaporized aerosol-forming substrate may escape from the cartridge through those portions of the susceptor which are not covered by the cartridge housing.

The cartridge housing 204 may also completely surround the susceptor 210. In this case, at least a portion of the cartridge housing 204 may be fluid permeable to allow vaporized aerosol-forming substrate to escape from the cartridge. Preferably, the complete cartridge housing 204 is fluid permeable as shown in **Fig. 9****.** Advantageously, this allows for maximum user experience.

Alternatively, the susceptor 210 may be contained in an impermeable cartridge housing 204 which completely surrounds the susceptor 210 as shown in **Fig. 10****.** Advantageously, this prevents the soaked susceptor from drying out. As shown in **Fig.** 11, at least a portion of the cartridge housing 204 may be removable or openable prior to engaging the cartridge with an aerosol-generating device, that is, prior to engaging the partially or completely freed susceptor with an aerosol-generating device. In the embodiment of Fig. 11, the cartridge housing 204 may be removable at an end face. Upon removal of the end face portion of the cartridge housing 204, the partially open cartridge may be engaged with an aerosol-generating device. During operation, vaporized aerosol-forming substrate may escape from the cartridge via the opened end face.

Likewise, the cartridge designs according to Figs. 4 and 5 or Figs. 6 and 7 may comprise a cartridge housing 204 which also covers the outer surface of the susceptor 210. The portion(s) of the cartridge housing 204 which cover the susceptor are to be removed or opened prior to engaging the cartridge with an aerosol-generating device. The removable or openable portion(s) of the cartridge housing may be considered to be a protective cover of the susceptor. The outer surface susceptor may be either flush with the outer surface of the remaining portions of the cartridge housing, or recessed such that the outer surface the cartridge housing is smooth.

**Fig. 12** and **Fig. 13** schematically illustrate a another cartridge design where the complete cartridge housing 204 is a protective cover or packing sleeve to be removed before engaging the remaining parts of the cartridge with an aerosol-generating device. Such a cover or sleeve may prove advantageous with regard to cartridges which essentially consist of a compact susceptor body (see Fig. 12) or which comprise a closed surface or hollow susceptor body encapsulating aerosol-forming liquid (see Fig. 13). In both embodiments, the susceptor body essentially constitutes the consumable aerosol-generating article to be engaged with an aerosol-generating device upon removal of a packing sleeve 204 that surrounds the article. As shown in Figs. 12 and 13, the surrounding packing sleeve 204 may be opened at an end face, allowing the susceptor body to be taken out. With regard to the susceptor design shown in Fig. 13, the closed susceptor surface may be considered as a remaining housing of those cartridge parts which are to be engaged with an aerosol-generating device.

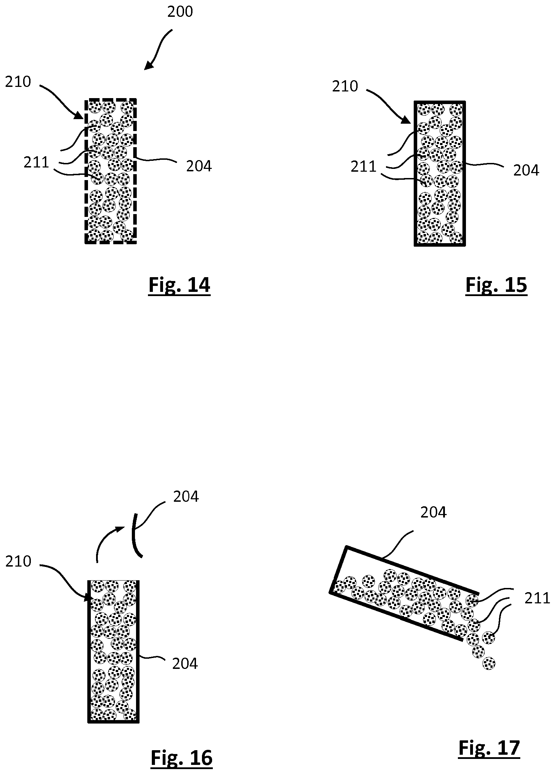

Instead of a unitary susceptor body, the susceptor may also comprise a plurality of susceptor elements 211. As shown in **Fig. 14****,** the susceptor elements may be individual susceptor pellets 211 soaked with aerosol-forming liquid forming a susceptor granulate. The susceptor elements 211 may be contained in a cartridge housing 204 at least a portion of which is fluid permeable. As example, a portion of the cartridge housing or the complete cartridge housing may be of mesh-like configuration, for example made of a stainless steel mesh. In Fig. 14, the complete cartridge housing 204 is fluid permeable. Advantageously, such a cartridge housing 204 holds the induvial susceptor elements together, but allows vaporized aerosol-forming substrate to escape from the cartridge.

Alternatively, the susceptor elements 211 may be contained in an impermeable cartridge housing 204 as shown **Fig. 15****.** Advantageously, this prevents the soaked pellets from drying out. As shown in **Fig. 16****,** at least a portion of the cartridge housing may be removable or openable such as to allow for taking out the individual susceptor elements (see **Fig. 17****)** which may be subsequently filled into a cavity of an aerosol-generating device. If the individual susceptor elements are to be received in an aerosol-generating device as bulk material without the cartridge housing, that is as loose items, the aerosol-generating device may comprise a receptacle for receiving and securely retaining the susceptor elements in the cavity. At least a portion of the receptacle may be fluid permeable to allow vaporized aerosol-forming substrate to escape form the receptacle. The receptacle may comprise a filling port. The filling port may be closable, for example by a lid or by a mouthpiece of the aerosol-generating device.

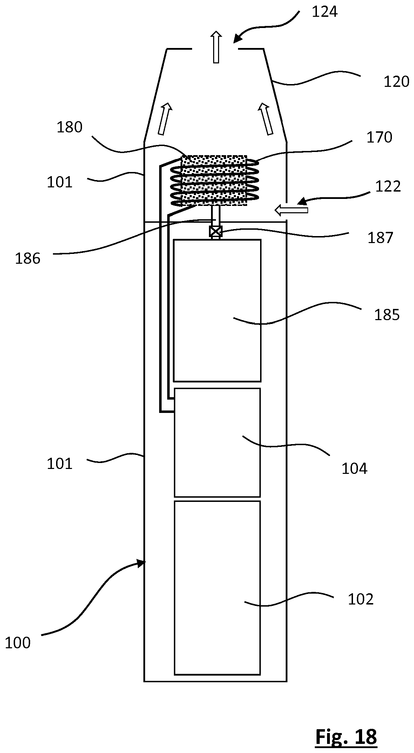

**Fig. 18** schematically illustrates a first embodiment of an aerosol-generating device 100 in accordance with another aspect of the invention. Instead of being engageable with a separate cartridge which contains a susceptor and aerosol-forming liquid to be heated, the aerosol-generating device 100 itself comprises a susceptor 180 according to the invention and as described herein, that is an internal susceptor 180 made of an open-porous ferrimagnetic ceramic material. Analogous to the aerosol-generating device shown in Fig. 1 and 3, the device 100 according to Fig. 13 comprises a main body having a main body housing 101 which contains a battery 102 and a control electric circuitry 104. The main body housing 101 defines a cavity 112 in which the internal susceptor 180 is arranged. The device 100 also includes a mouthpiece portion 120 comprising an outlet 124. A housing of the mouthpiece portion 120 and the main body housing 101 form together the housing of the device 100. The mouthpiece portion is removably connected to the main body. Air inlets 122 are defined in the main body housing. Within the cavity 112 is a helical induction coil 170. The coil 170 is operatively connected to the control electric circuitry 104 and surrounds the cylindrical susceptor body 180. When the electric circuitry 104 provides a high frequency oscillating current to the coil 170, an oscillating magnetic field is generated which passes through the susceptor 180. As a consequence, the susceptor 180 heats up due to hysteresis losses causing vaporization of aerosol-forming liquid held in the open-porous structure of the susceptor 180. The vaporized aerosol-forming material is entrained in an air flow which builds up when a user draws air though the air inlets 122 into the cavity 112 and the mouthpiece portion 120 and out of the outlet 124.

In the present example, the axial length extension of the helical coil 170 essentially corresponds to the axial length extension of the cylindrical susceptor 180. Of course, the coil 170 may also be configured such as to surround only an axial portion of the susceptor 180. Advantageously, the degree of overlap between the coil and the susceptor 180 may be used to pre-set the amount of aerosol-forming liquid to be heated and vaporized in order to optimize the user experience.

For repeatedly (re-)filling the susceptor 180 with aerosol-forming liquid, the aerosol-generating device 100 further comprises a tank 185 for aerosol-forming liquid. The tank may be replaceable or refillable. The tank 185 is arranged within the main body housing 101 of the device 100. The tank 185 is in fluid communication with the susceptor 180 via a fluid channel 186. A controllable valve 187 is arranged with the fluid channel 186. The valve 187 is operatively coupled to the electric circuitry 104 for controlling the shutting-off and opening of the valve. The aerosol-generating device 100 is configured such as to open the valve 186 only in an up-down or an overhead position of the device. Thus, soaking the susceptor 180 with aerosol-forming liquid from the tank 185 requires orientating the device 100 into this specific 'soaking' position. Advantageously, this reduces the risk of unwanted soaking or even oversoaking of the susceptor 180 beyond its capacity. Transfer of aerosol-forming liquid from the tank 185 to the susceptor 180 preferably occurs due to gravity. For detecting the respective position of the device, the device 100 may comprise a position sensor (not shown) as part of the electrical circuitry 104. In addition, the electrical circuitry 104 may be configured such as to disable the heating process in the 'soaking' position in order to prevent unintended gas formation. Furthermore, the electrical circuitry 104 may be configured such as to block the air path toward the outlet 124 during (re-)filling of the susceptor 180 in order to prevent unintended absorbing of aerosol-forming liquid by a user. For this, the device 100 may comprise a shutter (not shown). The device 100 may also be configured such as to enable heating of the susceptor 180 only in one or more predetermined positions of 'use'.

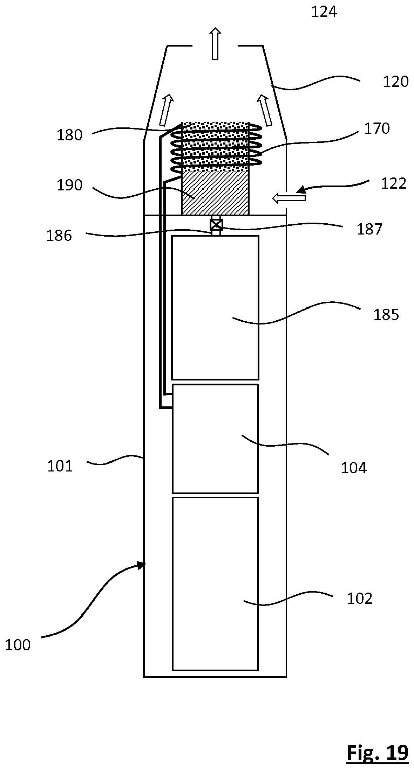

**Fig. 19** schematically illustrates a second embodiment of an aerosol-generating device 100 comprising an internal susceptor 180. This embodiment is essentially identical to the embodiment shown in Fig. 18. Therefore, in both embodiments identical features of the aerosol-generating device are denoted with identical reference numerals. In addition, the device 100 according to Fig. 19 comprises a liquid retention element 190 made of a high retention or high release material (HRM). The liquid retention element 190 serves a storage medium for aerosol-forming liquid in order to continuously soak the susceptor 180. For this, the liquid retention element 190 is in direct contact with the susceptor 180. Aerosol-forming liquid stored in the liquid retention element 190 is transferred by capillary action to the susceptor 180. The liquid retention element 190 is electrically non-conductive and paramagnetic and therefore inductively non-heatable. For this reason, the induction coil 170 only surrounds the susceptor 180. The liquid retention element is in fluid communication with the tank 185 via the fluid channel 186 to be (re-)filled with aerosol-forming liquid from the tank 185. Further advantages of the embodiment shown in Fig. 19 have been described with regard to the embodiment shown in Fig. 18 and will not be repeated.

## Claims

1. A cartridge (200) for use with an aerosol-generating device (100), the cartridge (200) consisting of an inductively heatable susceptor (210) soaked with aerosol-forming liquid (202),
wherein the inductively heatable susceptor (210) consists of an open-porous inductively heatable ceramic material for holding the aerosol-forming liquid (202) and for heating the aerosol-forming liquid (202) under the influence of an alternating electromagnetic field, and
wherein the susceptor (210) is a unitary body.

2. The cartridge (200) according to claim 1, wherein the open-porous inductively heatable ceramic material comprises or consists of at least one of:
- a manganese-magnesium ferrite;
- a nickel-zinc ferrite; or
- a cobalt-zinc barium ferrite.

3. The cartridge (200) according to claims 1 or 2, wherein the open-porous inductively heatable ceramic material has a porosity between 20 % and 60%.

4. The cartridge (200) according to any one of the preceding claims, wherein the open-porous inductively heatable ceramic material has a Curie temperature between 150 °C to 400 °C.

5. The cartridge (200) according to any one of the preceding claims, wherein the open-porous inductively heatable ceramic material is an electrically non-conductive material.

6. The cartridge (200) according to any one of the preceding claims, wherein the susceptor (210) forms a cartridge housing.

7. The cartridge (200) according to claim 6, wherein the cartridge (200) is a hollow cylinder comprising a circumferential wall and two end walls.

8. The cartridge (200) according to claim 7, wherein the circumferential wall and two end walls form the housing of the cartridge (200).

9. An aerosol-generating system (1) for generating an aerosol by inductively heating an aerosol-forming liquid (202), the system comprising an aerosol-generating device (100) and a cartridge (200) according to one of the claims 1 to 8 to be used with the aerosol-generating device (100), the aerosol-generating device (100) comprising:
a device housing (101) comprising a cavity (112) for receiving at least a portion of the cartridge (200);
an induction source within the device housing comprising an induction coil (170) for generating an alternating electromagnetic field;
wherein the susceptor (210) of the cartridge (200) is positionable in the cavity relative to the induction coil (170) such as to be inductively heatable by the alternating electromagnetic field.

## Patentansprüche

1. Patrone (200) zur Verwendung mit einer Aerosolerzeugungsvorrichtung (100), wobei die Patrone (200) aus einem induktiv heizbaren Suszeptor (210) besteht, der mit einer aerosolbildenden Flüssigkeit (202) getränkt ist,
wobei der induktiv heizbare Suszeptor (210) aus einem offenporigen, induktiv heizbaren Keramikmaterial zum Halten der aerosolbildenden Flüssigkeit (202) und zum Erwärmen der aerosolbildenden Flüssigkeit (202) unter dem Einfluss eines elektromagnetischen Wechselfelds besteht und
wobei der Suszeptor (210) ein einheitlicher Körper ist.

2. Patrone (200) nach Anspruch 1, wobei das offenporige, induktiv heizbare Keramikmaterial zumindest eines der Folgenden aufweist oder daraus besteht:
- ein Mangan-Magnesium-Ferrit,
- ein Nickel-Zink-Ferrit oder
- ein Kobalt-Zink-Bariumferrit.

3. Patrone (200) nach Anspruch 1 oder 2, wobei das offenporige, induktiv heizbare Keramikmaterial eine Porosität zwischen 20 % und 60 % besitzt.

4. Patrone (200) nach einem der vorhergehenden Ansprüche, wobei das offenporige, induktiv heizbare Keramikmaterial eine Curie-Temperatur zwischen 150 °C und 400 °C besitzt.

5. Patrone (200) nach einem der vorhergehenden Ansprüche, wobei das offenporige, induktiv heizbare Keramikmaterial ein elektrisch nichtleitendes Material ist.

6. Patrone (200) nach einem der vorhergehenden Ansprüche, wobei der Suszeptor (210) ein Patronengehäuse bildet.

7. Patrone (200) nach Anspruch 6, wobei die Patrone (200) ein Hohlzylinder ist, der eine Umfangswand und zwei Endwände aufweist.

8. Patrone (200) nach Anspruch 7, wobei die Umfangswand und zwei Endwände das Gehäuse der Patrone (200) bilden.

9. Aerosolerzeugungssystem (1) zum Erzeugen eines Aerosols durch induktives Erwärmen einer aerosolbildenden Flüssigkeit (202), wobei das System eine Aerosolerzeugungsvorrichtung (100) und eine Patrone (200) nach einem der Ansprüche 1 bis 8 aufweist, die mit der Aerosolerzeugungsvorrichtung (100) zu verwenden sind, wobei die Aerosolerzeugungsvorrichtung (100) aufweist:
ein Vorrichtungsgehäuse (101), das einen Hohlraum (112) zum Aufnehmen von zumindest einem Abschnitt der Patrone (200) aufweist,
eine Induktionsquelle innerhalb des Vorrichtungsgehäuses, die eine Induktionsspule (170) zum Erzeugen eines elektromagnetischen Wechselfeldes aufweist,
wobei der Suszeptor (210) der Patrone (200) in dem Hohlraum relativ zu der Induktionsspule (170) so positionierbar ist, dass er durch das elektromagnetische Wechselfeld induktiv heizbar ist.

## Revendications

1. Cartouche (200) destinée à être utilisée avec un dispositif de génération d'aérosol (100), la cartouche (200) étant constituée d'un suscepteur chauffable par induction (210) imbibé d'un liquide formant aérosol (202),
dans lequel le suscepteur chauffable par induction (210) est constitué d'un matériau céramique chauffable par induction à pores ouverts pour contenir le liquide formant aérosol (202) et pour chauffer le liquide formant aérosol (202) sous l'influence d'un champ électromagnétique alternatif, et
dans lequel le suscepteur (210) est un cops unitaire.

2. Cartouche (200) selon la revendication 1, dans laquelle le matériau céramique chauffable par induction à pores ouverts comprend ou est constitué d'au moins l'un parmi :
- un ferrite de manganèse-magnésium ;
- un ferrite de nickel-zinc ; ou
- un ferrite de baryum de cobalt-zinc.

3. Cartouche (200) selon la revendication 1 ou 2, dans laquelle le matériau céramique chauffable par induction à pores ouverts a une porosité entre 20 % et 60 %.

4. Cartouche (200) selon l'une quelconque des revendications précédentes, dans laquelle le matériau céramique chauffable par induction à pores ouverts a une température de Curie entre 150 °C et 400 °C.

5. Cartouche (200) selon l'une quelconque des revendications précédentes, dans laquelle le matériau céramique chauffable par induction à pores ouverts est un matériau électriquement non conducteur.

6. Cartouche (200) selon l'une quelconque des revendications précédentes, dans laquelle le suscepteur (210) forme un logement de cartouche.

7. Cartouche (200) selon la revendication 6, dans laquelle la cartouche (200) est un cylindre creux comprenant une paroi circonférentielle et deux parois d'extrémité.

8. Cartouche (200) selon la revendication 7, dans laquelle la paroi circonférentielle et les deux parois d'extrémité forment le logement de la cartouche (200).

9. Système de génération d'aérosol (1) pour générer un aérosol en chauffant par induction un liquide formant aérosol (202), le système comprenant un dispositif de génération d'aérosol (100) et une cartouche (200) selon l'une des revendications 1 à 8 à utiliser avec le dispositif de génération d'aérosol (100), le dispositif de génération d'aérosol (100) comprenant :
un logement de dispositif (101) comprenant une cavité (112) pour recevoir au moins une portion de la cartouche (200) ;
une source d'induction dans le logement de dispositif comprenant une bobine d'induction (170) pour générer un champ électromagnétique alternatif ;
dans lequel le suscepteur (210) de la cartouche (200) peut être positionné dans la cavité par rapport à la bobine d'induction (170) de manière à pouvoir être chauffé par induction par le champ électromagnétique alternatif.
